# EUROPÄISCHE PATENTANMELDUNG

(11) **EP 3 301 332 A1**
(43) Veröffentlichungstag der Anmeldung: **04.04.2018**
(21) Anmeldenummer: 17194274.1
(22) Anmeldetag: 30.09.2017
(51) Int. Cl.: F16J 3/04, A61M 1/00, B29C 47/02, F04B 43/08, F16L 51/02

(54) **RINGEBALG UND VERFAHREN ZUR HERSTELLUNG EINES RINGEBALGS**

(30) Priorität: 30.09.2016 DE 102016118636
(71) Anmelder: Eberhard Timm GmbH, 21614 Buxtehude (DE)
(72) Erfinder: Timm, Eberhard, 21614 Buxtehude (DE)
(74) Vertreter: Müller Schupfner & Partner Patent- und Rechtsanwaltspartnerschaft mbB

(57) **Zusammenfassung**

Die Erfindung bezieht sich auf einen Ringebalg umfassend eine Balghülle (1) mit einer elastischen Hüllenwand (3), an der in Radialebene steife Spannringe (10) festgesetzt werden, die durch Dehnen der Hüllenwand (3) Balgfalten (8) bilden und eine Blase- und/oder Saugvorrichtung umfassend den Ringebalg, sowie ein Verfahren zur Herstellung des Ringebalgs

## Beschreibung

Die Erfindung bezieht sich auf einen Ringebalg und eine Blase- und/oder Saugvorrichtung umfassend den Ringebalg, sowie ein Verfahren zur Herstellung eines Ringebalgs. Ein Ringebalg ist eine neue Kategorie von Faltenbalg, der ähnlich wie die Kategorie der Scheibenbälge unter den Oberbegriff der Faltenbälge subsummiert werden kann.

Ein Faltenbalg ist ein "ziehharmonikaartig" zusammenfaltbarer Schlauch aus einem Kunststoff- oder Ledermaterial, der zum Schutz über sich mechanisch ineinanderschiebende oder drehende, geschmierte Maschinenteile angebracht wird, um sie vor Fremdeinflüssen, insbesondere Verschmutzung zu schützen und gegenüber der Umgebung abzudichten, oder als ausziehbarer Verladeschlauch oder als Blase- und/oder Saugbalg eingesetzt wird.

Wird der Faltenbalg als Blase- und/oder Saugbalg betrieben, ist der Faltenbalg Bauteil in einer Vorrichtung zum Verdichten von gasförmigen Medien, zum Pumpen von flüssigen Medien oder zum Ansaugen von flüssigen oder gasförmigen Medien. Ein Blase- und/oder Saugbalg besteht aus einem verformbaren hohlen Körper, der i.d.R. mit zumindest einem Ventil ausgestattet ist. Durch das erste Ventil wird das gasförmige Medium beim Ausdehnen des Faltenbalgs eingesaugt, wenn der Faltenbalg als Saugbalg betrieben wird; in der anderen Richtung, d.h. beim Zusammendrücken des Faltenbalgs, sperrt das erste Ventil. Wird der Faltenbalg als Blasebalg betrieben, ist das erste Ventil so gestaltet, dass beim Zusammendrücken des Faltenbalgs das gasförmige Medium aus dem Faltenbalg durch das erste Ventil herausgeblasen wird und beim Ausdehnen des Faltenbalgs, das Ventil sperrt, so dass das gasförmige Medium nicht in umgekehrter Richtung in den Faltenbalg eindringen kann. Frühe Formen des Blase- und/oder Saugbalgs waren gänzlich ohne Ventile ausgebildet, und der Betreiber musste seine Hand vor die Öffnung setzen. Auch ist es möglich, dass der Faltenbalg kein wiederkehrendes Dehnen oder Zusammenzeihen ausführt, sondern lediglich eine Dehn- (ansaugen) oder eine Zusammenziehbewegung (herausblasen) und danach entleert bzw. wieder befüllt wird. In dieser Weise kann auch der erfindungsgemäße Ringebalg als Blase- und/oder Saugbalg betrieben werden. Ein Beispiel für eine mit einem Faltenbalg betreibbare Pumpe ist eine Membranpumpe.

Scheibenbälge werden aus flachen Scheiben hergestellt, die jeweils am inneren Umfang mit dem inneren Umfang der nächsten Scheibe und am äußeren Umfang mit dem äußeren Umfang der nächsten Scheibe verklebt sind. Hierzu werden einzelne Scheiben ausgestanzt, aufeinandergelegt und an den Innen- und Außenseiten vulkanisiert. Dadurch wird ein kleines Zusammendruckmaß erreicht. Gummischeibenbälge werden vor Auslieferung geschliffen. Je nach Länge und Anwendung werden zur Stabilisierung der Scheibenbälge Gleitbuchsen, Stützscheiben oder Drahtringe verwendet.

Multiflex-Bälge (auch Theku-Bälge genannt) sind aus thermosplastischem Kunststoff. Multiflex-Bälge sind sog. Tauchteile, für deren Herstellung eine Form erforderlich ist. Zur Stabilisierung können die Falten mit Drahtringen versteift werden.

Heute werden Faltenbälge häufig durch Spritzgießen hergestellt. Typische Materialien sind TPE, Polyurethan oder weich-PVC. Ob vulkanisierte Bälge, Gummischeibenbälge, robuste Bälge oder Gleitbahnschützer, die Auswahl an unterschiedlichsten Materialien und Ausführungsformen ist groß.

In der DE 102004037715 A1 ist ein Faltenbalg beschrieben, der dem Schutz eines Gelenkes einer Gelenkwelle dient. Der Faltenbalg besteht aus einer Hülle mit in Längsrichtung mit Abstand voneinander nach außen vorstehenden Außenfalten. Die Außenfalten sind mittels eingestülpter, elastischer Hüllenteile verbunden, die bei Knickbewegungen der Gelenkwelle auf der außen gelegenen Seite ausgezogen und der innen liegenden Seite zusammengedrückt werden. Eine solche Balgkonstruktion dient dem Schmutzabweisen; sie eignet sich nicht für axiale Pumpaktionen.

Aus der DE 202013008103 U1 ist ein Faltenbalg mit einer Mehrzahl von zueinander beweglichen Stützrahmen bekannt, wobei jeweils zwei benachbarte Stützrahmen über einen flexiblen Balgabschnitt mit Falte miteinander verbunden sind. Derartige Gleitbahnschützer werden an Maschinen zum Schutz gegen Verschmutzung der Gleitbahnen durch Staub, Späne, Kühlmittel usw. eingesetzt. Dem Menschen dienen sie zudem als Schutz vor Verletzungen beim Arbeiten an der Maschine (z. B. als Eingriffsschutz, Quetschschutz). Für Gleitbahnschützer sind etwaige innere Stütz- und Verstärkungsrahmen mit der Balgdecke thermisch verschweißt.

Die Faltenbälge nach dem Stand der Technik sind bezogen auf den Umfang der Faltkanten, sowohl der inneren Faltenkante als auch der äußeren Faltenkante, nicht dehnbar und der Umfang der Faltkanten bleibt immer gleich, egal ob sich der Faltenbalg "ziehharmonikaartig" zusammenfaltet oder "ziehharmonikaartig" auseinanderfaltet oder dieser in Bezug auf die Mittelachse geknickt wird. Die inneren Faltkanten herkömmlicher Faltenbälge wirken dem Auseinanderfalten entgegen, weil sie als Knickkanten eine Materialverstärkung darstellen und die Knickkante immer in die Ruhelage strebt, d.h. in Richtung eines Winkels von 0°. Unter dem Eigengewicht falten sich herkömmliche Faltenbälge nicht gleichmäßig auf, sondern oben stärker, wo mehr Schwerkraft wirkt und unten weniger.

Es ist Aufgabe der Erfindung, eine neue Kategorie von Faltenbalg für eine Vorrichtung zum Be- und Entlüften mit einem gasförmigen Medium zu schaffen, der bei einem einfachen Aufbau eingebaut zum Belüften axial zusammendrückbar und zum Entlüften des Gebrauchsartikels auseinanderziehbar ist. Es soll also ein Faltenbalg für eine Pump- und Absaugfunktion sein, insbesondere eine Absaugfunktion.

Weiterhin sind herkömmliche Faltenbälge häufig aus einem Gummimaterial, das langanhaltend einen starken Geruch entfaltet. Dieser Geruch ist in der Medizintechnik und insbesondere in Räumen in denen sich Personen auf Dauer aufhalten nicht einsetzbar, wie z.B. Patienten in einem Behandlungsraum oder Krankenzimmer. Es gilt daher Bälge zugänglich zu machen, die aus einem besser verträglichem Material bestehen, dass z.B. keinen unangenehmen Geruch absondert.

Die gestellte Aufgabe ist erfindungsgemäß durch den Gegenstand der unabhängigen Patentansprüche gelöst. Vorteilhafte Ausgestaltungen sind Gegenstand der Unteransprüche oder nachfolgend beschrieben.

Der Ringebalg kann zum Be- und Entlüften von Gebrauchsartikeln mit einem gasförmigen Arbeitsmedium, vorzugsweise Luft, zum Belüften aus einer ausgezogenen Arbeitsstellung während eines Pumpvorganges zusammengedrückt werden oder zum Entlüften aus einer zusammengedrückten Arbeitsstellung während eines Absaugvorganges ausgezogen werden.

In einer bevorzugten Ausführungsform entspricht das Absaugvolumen etwa dem Volumen, das durch Ausziehen des Ringebalgs entsteht, vorzugsweise Ausziehen durch Anwendung der Schwerkraft auf das untere Balgende unter Verwendung eines den Durchlass des einströmenden Mediums begrenzenden Ventils.

Ausgangsbasis für den Ringebalg ist eine im Umfang elastische Balghülle, die nach einer Ausgestaltung ausgedehnt im Wesentlichen zylinderförmig ist, abgesehen von den Faltenkanten. Die Balghülle kann z.B. aus einem Endlosschlauch bestehen. Herkömmliche Faltenbalge oder gespritzte Balge weisen eine starre Balghülle auf, die sich nur entlang der Faltkanten bewegt und wo die Hüllenwand nicht elastisch gedehnt ist und daher mit einer Kraft nach innen strebt.

An der Hüllenwand der Balghülle sind zur Herstellung des Ringebalgs längs der Balghülle im Abstand der zu bildenden Balgfalten und zu deren Ausbildung in Radialebenen steife Spannringe platziert, die durch Dehnen der Hüllenwand die Balgfalten des Ringebalgs bilden. Bei der Montage werden die Spannringe in die Balghülle eingeführt oder auf diese aufgesetzt und dabei in die gewünschten Positionen gebracht. Die Spannringe dehnen die Balghülle im Bereich der Balgfalten und bilden die äußere Faltenkante aus. Damit ist die zunächst funktionslose Balghülle mit wenigen Umbauten in einen Ringebalg umgewandelt.

Der Ringebalg wird vorzugsweise vertikal eingehängt betrieben. Die Balghüllenteile zwischen den Balgfalten haben dort, wo sie nicht durch Spannringe gedehnt sind, einen nach innen eingeschnürten Verlauf, der sich bei den Pumpvorgängen ändert und zwar zwischen eingeschnürter und zusammengefalteter Gestalt und ausgedehnter Gestalt, in der sich die Einschnürungen glätten und ausdehnen in Richtung auf den Umfang der Spannringe. Der Ringebalg hat eine gute Formstabilität. Dazu kommt eine gute Umfangsdehnbarkeit der Zonen zwischen den Balgfalten.

Der nach innen eingeschnürte Verlauf stellt die innere Faltenkante dar, ohne dass diese tatsächlich eine Kante ausbildet bzw. ausbilden muss, vielmehr wird vorzugsweise ein runder Bogen, z.B. in Parabelform, ausgebildet. Die gute elastische Umfangsdehnbarkeit ist entscheidend während eine zusätzliche Dehnbarkeit in der Länge, d.h. in Richtung der Achse des Ringebalgs nicht obligatorisch ist und nur eine Option ist.

Nach einer weiteren Ausgestaltung der Erfindung ist vorgesehen, dass die elastische Balghülle in den Radialebenen der auszubildenden Falten axial mit auf Abstand paarweise angeordneten Wülsten oder Noppenreihen oder einer Nut versehen ist, die bei den Paaren aufeinander zu aus der Hüllenwand hervorstehen und rundum laufen.

Nach einer weiteren Ausgestaltung der Erfindung ist vorgesehen, dass die Spannringe von den Wülsten oder Noppenreihen innen hintergriffen werden und damit durch Einklemmen fixiert sind zwischen den Wülsten oder Noppenreihen und Hüllenköpfen, die die Spannringe außen umgreifen und die beim Ausziehen und Dehnen der Hüllenwand zur Bildung der Balgfalten entstanden sind.

Es ist vorgesehen, dass die Spannringe stets einen größeren Umfang als die ungedehnte Balghülle haben, vorzugsweise einen zumindest 5 % größeren Umfang, vorzugsweise einen zumindest 10%, insbesondere mindestens 20% größeren Umfang.

Nach einer weiteren Ausgestaltung der Erfindung ist vorgesehen, dass die Wülste oder Noppenreihen aus der Hüllenwand nach außen hervorstehen für außen auf die Balghülle aufgesetzte Spannringe, die von den Wülsten oder Noppenreihen außen übergriffen und damit die Spannringe durch Einklemmen fixiert werden zwischen den Wülsten oder Noppenreihen und den die Spannringe innen umgreifenden Hüllenwandköpfen.

Es ist vorgesehen, dass der Ringebalg mindestens drei Spannringe aufweist, vorzugsweise mindestens 6.

Nach einer weiteren Ausgestaltung der Erfindung ist vorgesehen, dass die Spannringe den Anforderungen entsprechend kreisrund oder von der Kreisform abweichend, beispielsweise oval geformt sind. Die kreisrunde Zylinderform ist die einfachste; äußere Zwänge können aber beispielsweise abweichende Formen nötig machen.

Die Spannringe sind entweder aus Draht gefertigt, etwa rostfreiem V2A-Stahl, oder aus Kunststoff, etwa faserverstärkten Kunststoff. Zum nachträglichen einsetzen in den Innenumfang kann es erwünscht sein den Ring zu einer Acht zu falten und den Ring an seinem Bestimmungsort zu entfalten. Ggf. sind auch Scheiben möglich.

Nach einer weiteren Ausgestaltung der Erfindung ist vorgesehen, dass die Spannringe alternierend im Umfang variieren, so dass jeweils ein größerer einem kleineren Spannring auf oder in dem Balg folgt. Dies hat den Vorteil, dass beim Zusammendrücken des Ringebalgs der kleinere Spannring in dem Umfang des größeren aufgenommen wird und sich so Balghöhe einsparen lässt.

Denkbar sind auch andere ähnliche Anordnungen, wo zum Beispiel zunächst auf einen größeren Spannring drei kleinere Spannringe folgen, wobei jeder der kleineren Spannringe seinerseits einen benachbarten Ring in seinem Umfang aufnehmen kann, bis schließlich wieder ein größerer Spannring folgt, der dem erstgenannten größeren Spannring gleicht und dem wiederum drei kaskadenartig kleiner werdende kleinere Spannringe folgen usw.

Nach einer bevorzugten Ausführungsform sind die Spannringe ausschließlich dazu vorgesehen, die Balghülle im Umfang zu strecken und nicht bzw. nicht zusätzlich dazu vorgesehen, den Umfang der Balghülle einzuengen. Einengungen der Balghülle wie innere Balgfalten bilden sich vorzugsweise ausschließlich durch die elastische Rückstellkraft der Hüllenwand aus.

Auch ist es möglich neben einem inneren Spannring weiterhin jeweils einen äußeren Ring oder bevorzugt mehrere Ringabschnitte von außen auf den inneren Spannring aufzusetzen, um den inneren Spannring klammerartig festzusetzen, wobei der äußere Ring oder Ringabschnitt über die Hüllenwand greift und den inneren Spannring umgreift. Der äußere Ring oder die Ringabschnitte können aus Kunststoff oder Metall sein.

Die Balghülle ist aus einem elastischen reißfesten und im Wesentlichen gasdichten Material gebildet. Geeignete Materialien sind etwa Silicon-Kunststoffe. Ein Beispiel hierfür ist Vinlymethylpolysiloxan oder LDPE, z.B. mit einer Dicke von 25 µm (mü).

Die Balghülle hat vorzugsweise eine Materialstärke von z.B. ca. 0,01 bis 2 mm, insbesondere 0,05 bis 1 mm und besonders bevorzugt von 0,2 bis 0,8 mm.

Der Ringebalg nach der vorliegenden Erfindung ist einfacher und leichter als andere Faltenbälge. Das Formen des Ringebalgs mit Spannringen bringt eine deutliche Material- und Kostenersparnis, insbesondere gegenüber Scheibenbälgen. Im Vergleich zu herkömmlichen gespritzten Faltenbälgen und Scheibenbälgen ist bei gleichem Volumen, der Ringebalg in der Hüllenwandfläche ca. 40% kleiner, was sich damit erklärt, dass er konstruktiv weniger Balgfalten haben kann. Dadurch kann die Bauhöhe geringer sein. Die Elastizität des Ringebalgs ist größer als die durch Vulkanisieren hergestellter Scheibenbälge.

Der Ringebalg ist beispielsweise als Absaugvorrichtung geeignet für zu belüftende Absorptionsartikel wie Windeln, Windelhosen oder Binden, wie dies in der WO 2014180461 A1 beschrieben ist. Eine Vorrichtung in der der Ringebalg einsetzbar ist, ist z.B. in der DE 102014101922 B3 beschrieben.

Interessant ist auch, dass sich der Ringebalg in vertikaler Richtung gegen eine Federspannung auszieht, die der Dehnung der Innenhülle im Bereich der Einschnürungen entspricht. Die Einschnürungen, die die inneren (runden) Falten bilden, dehnen sich aus, bis sie dem Umfang der äußeren Falten im Bereich der Spannringe ähneln.

Der Ringebalg mit eingesetzten Spannringen kann z.B. hergestellt werden, indem die Balghülle in eine Zylinderform gehängt und oben mit einem Expander eingespannt wird. Der Balg wird unten ebenfalls mit einem Expander eingespannt und die Balghülle durch Druck plan an die Zylinderinnenwand angelegt. Im oberen Expander ist ein Spritzautomat integriert, der horizontal und vertikal beweglich ist und auf die Höhe des Spannringes ausgerichtet wird. Die Fixierung der Spannringe auf den Balg erfolgt wie folgt. Das Distanzteil mit der Spritzdüse fährt aus dem Spritzautomaten bis an den Spannring. Bei einer 380° Horizontaldrehung, spritzt der Automat von innen über den Spannring einen dünnflüssigen reaktiven Kunststoff, der den Ring und den Balg beidseitig verbindet. Nach Beendigung des ersten Arbeitsteils, zieht sich das Distanzteil wieder zurück und die Welle fährt danach den Spritzautomaten einen Spannring tiefer. Danach widerholt sich der nächste Arbeitsteil, bis zum letzten Spannring. Zum Abschluss werden beide Expander gelöst und entfernt, und der Ringebalg aus der Zylinderform entnommen.

Die Erfindung wird anhand der Zeichnung näher erläutert, ohne auf diese beschränkt zu sein. Es zeigen:
Fig.1 eine Balghülle, die durch Ansetzen von Spannringen im Bereich der zu bildenden Falten des Balgs zu einem Ringebalg geworden ist,
Fig. 2 einen Spannring, wie er in die Balghülle nach Figur eingesetzt ist,
Fig. 3 den vor einem Auseinanderfalten zusammengelegten und gefalteten Ringebalg mit von innen eingesetzten Spannringen, die die Balghülle im Bereich der Falten ausdehnen und damit die Balgfalten bilden,
Fig. 4 eine vergrößerte Darstellung der Ausbildung einer Balgfalte des Ringebalgs nach Fig. 3 mit einem an der Balghülle vorgesehenem Paar von Wülsten, die in Längsrichtung der zu bildenden Balgfalte in Radialebenen an der Hüllenwand umlaufen, wobei auf die Hüllenwand nach Fig. 3 in der Nut, die sich zwischen den Wülsten verläuft, jeweils Spannringe aufgesetzt sind, die die Hüllenwand zum Bilden der Balgfalten ausziehen und dehnen und wobei die Wülste die hintergriffenen Spannringe in den Radialebenen der Balgfalten fixieren,
Fig. 5 den Ringebalg noch ohne Spannringe im hängend gestreckten Zustand, wobei die Ringbalghülle drucklos vertikal am oberen Anschlussring hängt. Der untere Teil mit Anschlussring ist im Querschnitt reduziert,
Fig. 6 bis 8 einen Ringebalg-Sauger mit dem erfindungsgemäßen Ringebalg, und
Fig. 9 bis 11 ein Verfahren zur Herstellung eine Ringebalgs.

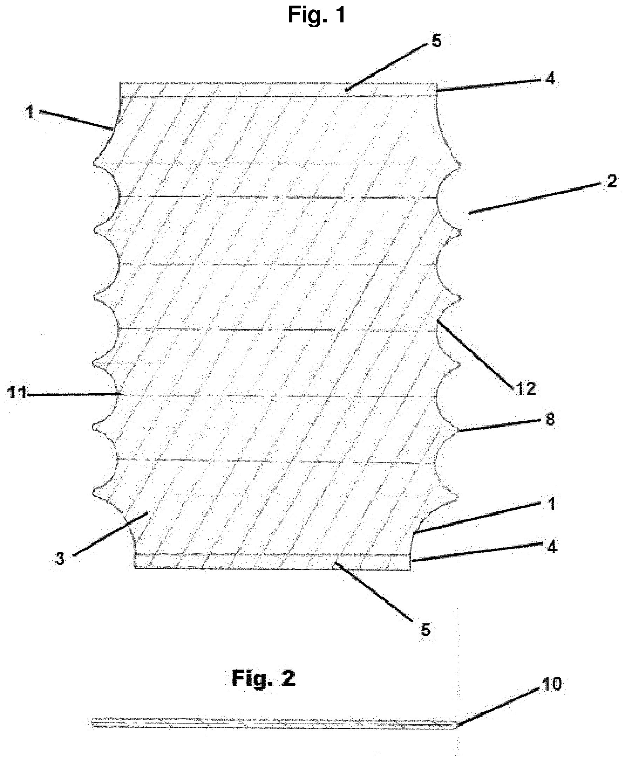

Fig. 1 zeigt eine Balghülle 1, die zur Bildung eines zylindrischen Ringebalgs 2 nach der Erfindung benutzt wird. Die Balghülle 1 ist vertikal hängend eingespannt; Die Hüllenwand 3 der Balghülle 1 ist flexibel, gut dehnbar und im verwendeten Umfang reißfest. An den oberen und unteren Balgenden 4 sind Anschlussringe 5 zum Anschluss an einen nicht dargestellten Pumpmechanismus vorgesehen. Die Anschlussringe 5 bestehen aus einem steifen Material, wie beispielsweise Metall, vorzugsweise korrosionsfreiem Stahl.

Die Anschlussringe 5 umfassen die Hüllenwand 3 an den Balgenden 4 und legen diese am oberen bzw. unteren kreisrunden Umfang des nicht dargestellten Deckels fest. In die Balghülle 1 sind sechs kreisrunde Spannringe 10 eingesetzt.

Fig. 2 zeigt einen kreisrunde Spannring 10, der in die Balghülle 1 nach Figur 1 eingesetzt ist. Der Durchmesser des Spannrings 10 bzw. sein Umfang ist so groß, dass der Spannring die Balghülle 1 im Bereich der Balgfalten 8 deutlich ausdehnt. Damit bilden die Spannringe die Balgfalten 8 des Ringebalgs 2 nach Fig.1.

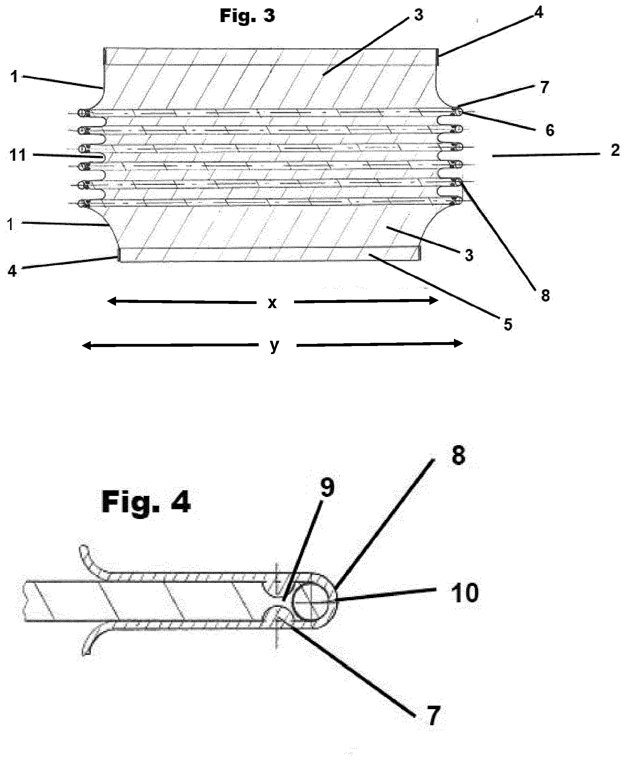

Fig.3 zeigt die zu einem Ringebalg 2 gewordene Balghülle 1. In die Balghülle 1 sind kreisrunde Spannringe 10 eingesetzt. Ihre radiale Ausdehnung ist so groß, dass sie die Balghülle 1 im Bereich der Balgfalten 8 deutlich ausdehnen. Damit bilden sie die äußeren Balgfalten 8 des Ringebalgs 2.

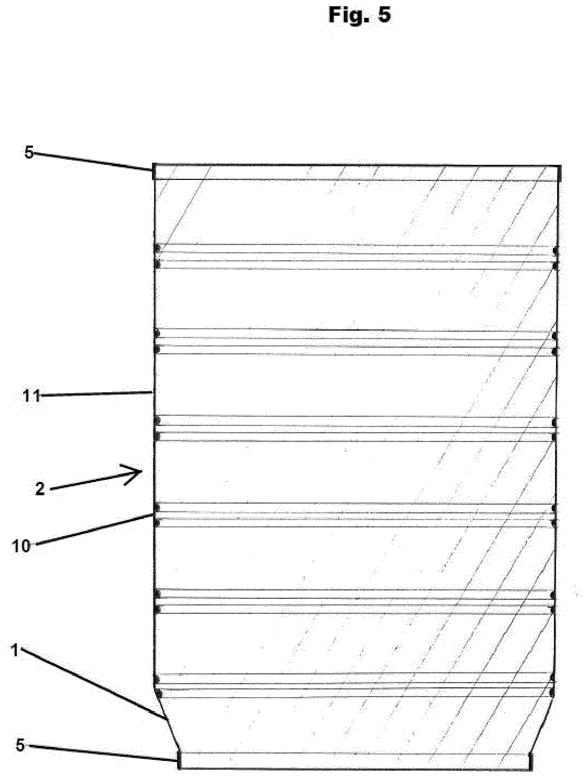

Durch das Auffalten der Falten 8 vergrößert sich der Durchmesser x im Bereich der inneren Balgfalten 11 in Richtung auf den Wert y. Der Außenumfang des Ringebalgs nähert sich einer zylindrischen Form. Das untere Balgende 4 ist in Fig. 3 und Fig. 5 im Durchmesser gegenüber dem Ausgangsdurchmesser x etwas verkleinert.

Fig. 4 zeigt einen Ausschnitt aus der Balghülle 1 im Bereich der Balgfalten 8. Man sieht in Fig. 4 in Radialebene 6 innen an die Hüllenwand 3 der Balghülle 1 angespritzte Erhebungen, die Wülste 7 bilden. Die Wülste 7 sind paarweise vorgesehen. Die Paare haben zu benachbarten Wulstpaaren Abstände, die die Abstände der zu bildenden Balgfalten 8 des Ringebalgs 2 bestimmen. Die Wülste 7 laufen rund um die Balghülle 1. In einer Variante ist vorgesehen, sie zu unterbrechen, sodass aus den Wülsten 7 Noppenreihen entstehen. Fig.4 zeigt anhand eines vergrößerten Ausschnittes durch den zusammengefalteten Ringebalg 2 nach Fig. 3 den konstruktiven Aufbau der Balgfalten 8.

Mit dem Spannring 10 ist die Balghülle 1 im Bereich der Balgfalten 8 gedehnt und ausgezogen. Die ausgezogenen Teile 12 im Bereich der inneren Balgfalten 11 der Balghülle 1 liegen seitlich am Spannring 10 an. Der im Querschnitt runde Spannring 10 wird hintergriffen von den Wülsten 7 der Balghülle 1.

Der Spannring 10 ist also innen eingeklemmt zwischen den Wülsten 7, die dazwischenliegend eine Nut ausbilden. Es ist möglich, die Konstruktion dadurch zu verändern, dass die Spannringe 1 von außen auf die Balghülle 1 aufgesetzt werden. In diesem Fall sind auch die Wülste 7 außen angespritzt (nicht dargestellt).

Fig. 5 zeigt den Ringebalg 2 nach Fig. 3 in vertikal hängendem Zustand ohne Spannringe. Die Balghüllenteile 12 zwischen den Balgfalten 8 haben aufgrund der fehlenden Spannringe 10 noch keinen nach innen eingeschnürten Verlauf. Die Balghülle hat im Wesentlichen eine zylindrische Form.

Alle Spannringe 10 eines Ringebalgs 2 sind gleichgeformt. Von Modell zu Modell können sie aber auch verschieden sein. So können die Spannringe 10 auch oval oder elliptisch sein. Nur Spannringe 10 mit eckigen Formgebungen sind i.d.R. weniger bevorzugt.

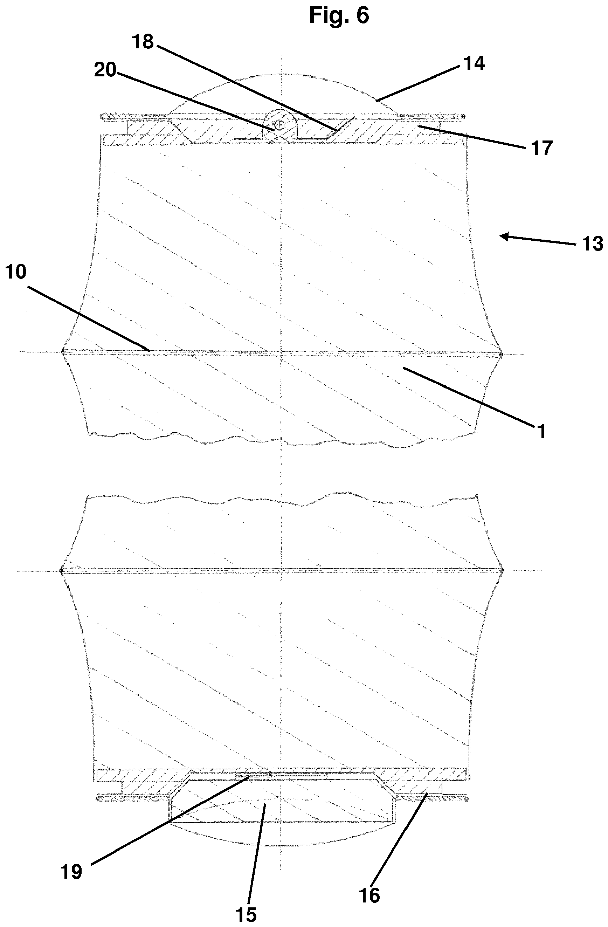

In Fig. 6 ist ein Absauger umfassend einen Ringebalg in hängender, mit einem Gewicht belasteter Form, gezeigt, um bei seiner Entfaltung im inneren einen gewollten Unterdruck zu erzeugen. Der Ringebalg-Sauger 13 ist in der Darstellung voll ausgestreckt. Er besitzt 3 oder mehr Spannringe 10.

Eine preislich besonders günstige Ausführungsform, die auch als Einweg-Wegwerfartikel eingesetzt werde kann, besteht aus einem dehnbarem Folienschlauch z.B. LDPE 25 µm. Die Spannringe 10 können eingesetzte Ringe aus Kunststoff oder Stahl sein, oder sie werden im Spritzverfahren hergestellt. Die Funktion des Ringebalg-Saugers 13 ist wie folgt: Der Sauger wird mit dem Griff 14 an ein Stativ gehängt und die Balghülle 1 an einen Schlauchanschluss 20 gesteckt. Danach wird zur Erzeugung des Unterdrucks ein Gewicht 15 unter den Boden 16 geheftet. Mit der werksseitig über ein Drosselventil eingestellten Absaugmenge von ca. 200 ml / min beginnt die Funktion. Wenn der Absaugprozess beendet ist, bzw. der Sauger voll ausgesteckt ist, werden der Schlauch vom Schlauchanschluss 20 und das Gewicht 15 vom Boden 16 abgezogen. Der Einmal-Sauger wird mit dem Griff 4 senkrecht zur Entsorgung getragen und durch Aufziehen der Volumenöffnung 18 entleert. Dann verkleinert sich der Ringebalg-Sauger 13 wieder.

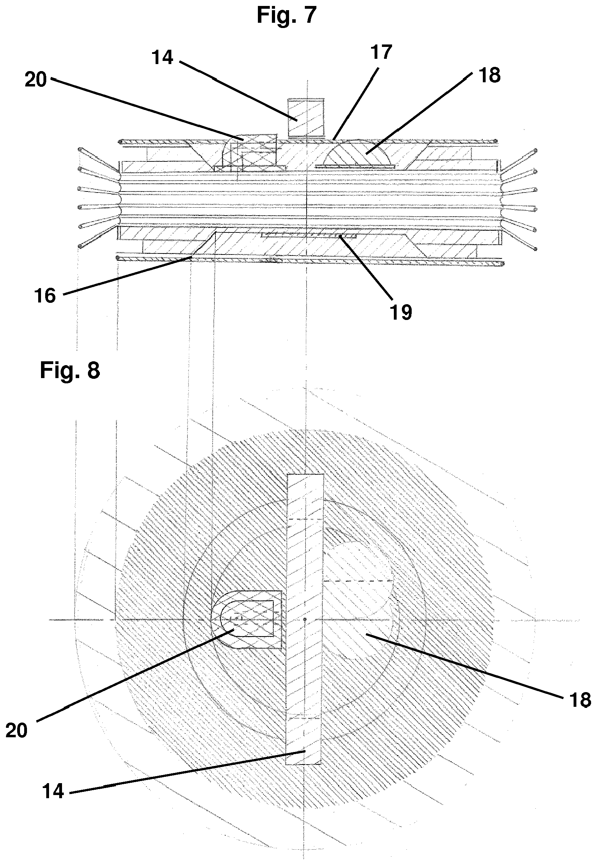

In Fig. 7 ist der Ringebalg-Sauger 13 in der Handelsform zusammengefaltet gezeigt mit Boden 16 und Deckel 17 aus Alu-Folie. In einer Vertiefung des Deckels ist ein Schlauchanschluss 20 mit Öffnung in den Balg und eine noch verschlossene Folienöffnung 18, die zur Entsorgung des Ringebalg-Saugers benötigt wird. Zur Handhabung ist ein Griff 14 angebracht, der auch zum Aufhängen an ein Stativ dient. In der Vertiefung des Bodens ist eine Seite eines Klettbandes 19 angebracht, an dem das Gewicht halt findet.

Fig. 8 zeigt den Ringebalg-Sauger von oben. Er ist als einmal zu verwendende Handelsware gedacht. Zu ihrem Gebrauch, werden separat zur ständigen Wiederverwendung, ein Set bestehend aus Stativ und Gewicht, z.B. ca. 250 Gramm, mit Gegenseite des Klettbandes angeboten.

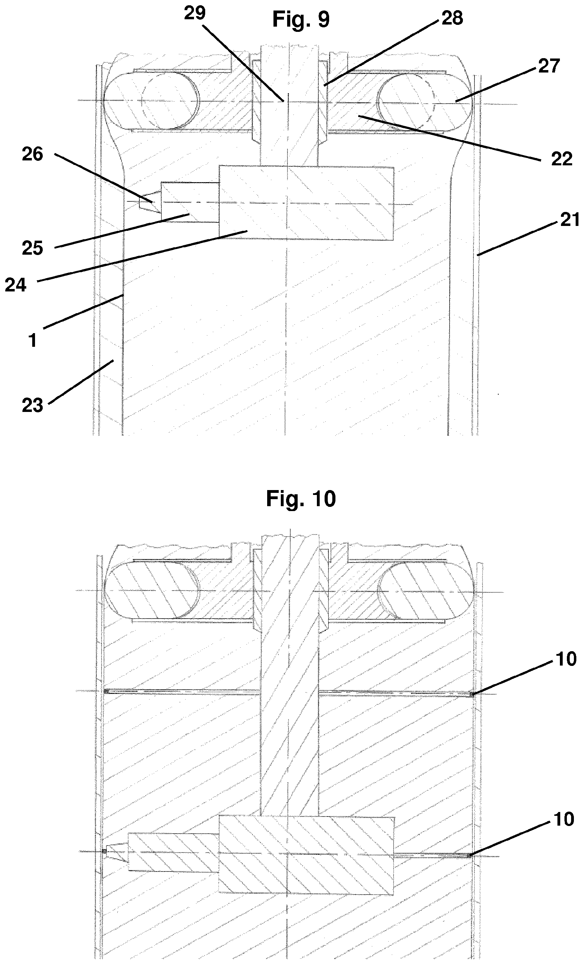

Nachfolgend wird ein Verfahren zur Herstellung des Ringebalges 2 erläutert. Eine in der Länge dehnbare Balghülle 1, z.B. aus einem Folienschlauch mit einer Stärke von 0,02 - 1,0 mm, wird in einen Zylinder 21 mit größerem Umfang gehängt und oben mit einem Expander 22 und Dehnungsringen 27 luftdicht eingespannt. Im oberen Expander 22 ist ein Spritzautomat 24 integriert, der horizontal und/oder vertikal beweglich ist und über eine Welle 29 in der Höhe verfahrbar ist. Unten (nicht gezeigt) wird wie oben, bei Straffung der Balghülle 1 ein zweiter Expander eingesetzt. Der zweite Expander hat ein Ventil für den Druckausgleich. Über dieses Ventil wird Druck im Inneren der Balghülle 1 aufgebaut. Dabei wird die Luft zwischen Außenfläche der Balghülle 1 und Zylinder 21, nach unten verdrängt (Fig. 9) und kann durch den Restspalt 23 entweichen.

Danach liegt der Balghülle 1 geweitet an der Innenwand des Zylinders 21 (Fig. 10) und der zweite Expander wurde ebenfalls luftdicht gegen die Form gepresst. Die Herstellung der Spannringe 10 auf der Innenseite der Balghülle 1 erfolgt wie folgt: Der auf Höhe gesteuerte Spritzautomat 24 wird mit der Welle 29 auf die Höhe gefahren. Die Welle 29 läuft in einer Wellenmanschette 28. Das Distanz-Teil 25 mit der Spritzdüse 26 fährt gesteuert auf Abstand vor die Innenwand der Balghülle 1. In einer oder mehreren Lagen, trägt der Spritzautomat 24 bei einer oder mehreren 360° Horizontaldrehungen einen aushärtenden, auf dem Untergrund haftenden Ring aus Kunststoff auf. Fig. 10 zeigt die Herstellung des Spannringes der zweiten Ebene von oben. Der Spannring 10 der ersten Ebene ist fertig gestellt.

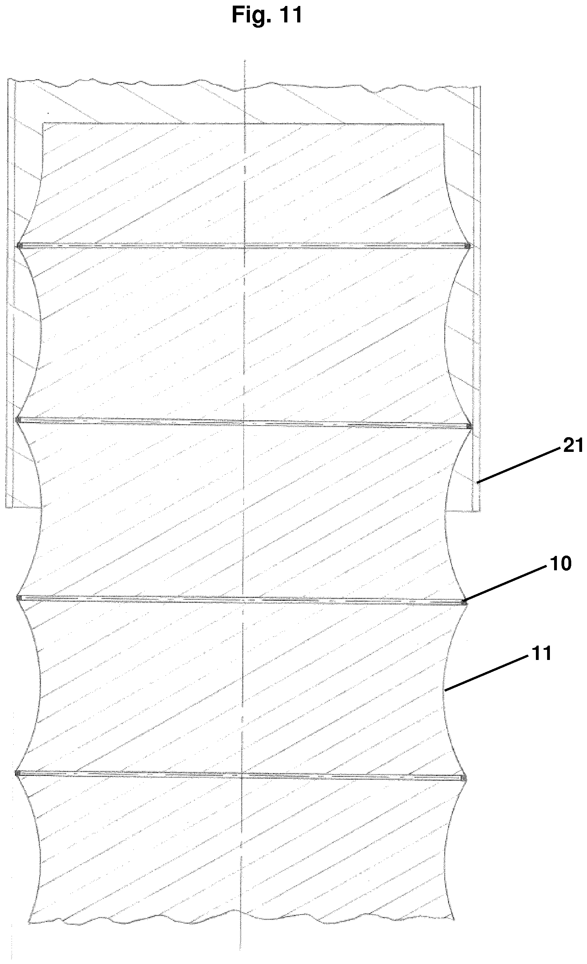

In Fig. 11 sind die zwei Expander 22 entfernt und der Druck ist ausgeglichen. Die Verformung der Balghülle zwischen den Spannringen 10, ist auf das ursprüngliche Maß zurückgestellt (innere Balgfalten 11). Die Aushärtung des Materials der gespritzten Ringe führt zu einer minimalen Schrumpfung, dadurch lässt sich der fertige Balg einfach aus dem Zylinder entnehmen.

**Bezugszeichenliste:**

| | | | |
|---|---|---|---|
| (1) | Balghülle | (16) | Boden |
| (2) | Ringebalg | (17) | Deckel |
| (3) | Hüllenwand | (18) | Volumenöffnung |
| (4) | Oberes und unteres Ende der Balghülle | (19) | Klettband |
| (5) | Anschlussringe | (20) | Schlauchanschluss |
| (6) | Radialebenen der Balgfalten | (21) | Zylinder |
| (7) | Erhebungen in Form von Wülsten | (22) | Expander |
| (8) | Balgfalten | (23) | Restspalt |
| (9) | Nut | (24) | Spritzautomat |
| (10) | Spannringe | (25) | Distanzteil |
| (11) | innere Balgfalte | (26) | Spritzdüse |
| (12) | Balghüllenteile zwischen Balgfalten | (27) | Dehnungsring |
| (13) | Ringebalg-Sauger | (28) | Wellenmanschette |
| (14) | Griff | (29) | Welle |
| (15) | Gewicht | | |

## Patentansprüche

1. Ringebalg (2) umfassend eine Balghülle (1), die aufweist eine elastische schlauchförmige Hüllenwand (3), an der in Radialebene zumindest drei steife Spannringe (10) festgesetzt sind, die durch Dehnen der Hüllenwand (3) im Umfang herausstehende Balgfalten (8) bilden, wobei die Spannringe (10) einen größeren Durchmesser als die ungedehnte elastische Hüllenwand (3) haben und die Hüllenwand (3) zwischen den Balgfalten (8) zumindest im zusammengefalteten Zustand elastisch zurückspringt.

2. Ringebalg nach Anspruch 1, wobei zum Bestimmen der Sitzposition der Spannringe (10) an der Hüllenwand (3) in den Radialebenen (6) der zu bildenden Balgfalten (8) Erhebungen (7) oder Vertiefungen an der Hüllenwand (3) vorgesehen sind, die die Spannringe (10) verrutschsicher festlegen.

3. Ringebalg nach Anspruch 2, wobei die Hüllenwand (3) jeweils in den Radialebenen (6) der auszubildenden Balgfalten (8) aufweist:
- auf Abstand angeordnete und umlaufende Wülste (7) oder Noppenreihen, die als Erhebungen jeweils beidseitig der Spannringe (10) aus der Hüllenwand (3) hervorstehen, oder
- eine umlaufende Nut (9), die jeweils einen Spannring in der Nut (9) aufnimmt.

4. Ringebalg nach Anspruch 2, wobei die Wülste (7) oder Noppenreihen oder die Nut aus der Hüllenwand (3) nach innen hervorstehen für innen auf der Hüllenwand (3) aufgesetzte Spannringe (10).

5. Ringebalg nach Anspruch 2 oder 3, wobei die Wülste (7) oder Noppenreihen oder die Nut aus der Hüllenwand (3) nach außen hervorstehen für außen auf der Hüllenwand (3) aufgesetzte Spannringe (10), die von den Wülsten (7) oder Noppenreihen oder der Nut außen übergriffen werden und damit durch Einklemmen fixiert sind und die Spannringe vorzugsweise ein- bzw. aufgeklebt sind.

6. Ringebalg nach Anspruch 1 oder 2, wobei die Spannringe (10) innen und/oder außen auf der Hüllenwand (3) aufgeklebt sind, aufgespritzt sind oder thermisch aufgesiegelt sind oder ein innerer Spannring jeweils von einem einen äußeren Ring oder mehreren Ringabschnitte, die außen aufgesetzt werden klammerartig festgesetzt wird, wobei der äußere Ring oder Ringabschnitt über die Hüllenwand greift.

7. Ringebalg nach einem der vorhergehenden Ansprüche, wobei der Ringebalg (2) mit mindestens sechs von Spannringen (10) gebildeten Balgfalten (8) versehen ist.

8. Ringebalg nach einem der vorhergehenden Ansprüche, wobei die Spannringe (10) kreisrund oder oval geformt sind.

9. Ringebalg nach einem der vorhergehenden Ansprüche, wobei die Spannringe zumindest teilweise alternierend im Umfang variieren, insbesondere so dass ein größerer einem kleineren Spannring auf oder in dem Balg folgt.

10. Ringebalg nach einem der vorhergehenden Ansprüche, wobei die Hüllenwand aus einem elastischen Schlauch, vorzugsweise aus einem Silicon-Kunststoff, gebildet ist mit einer Materialstärke von 0,01 bis 2 mm.

11. Blase- und/oder Saugvorrichtung umfassend den Ringebalg nach zumindest einem der vorhergehenden Ansprüche und weiterhin zumindest ein Ventil.

12. Saugvorrichtung nach Anspruch 11, wobei das Ventil ein Nadelventil ist und der Ringebalg als Saugbalg ausgebildet ist.

13. Saugvorrichtung nach Anspruch 11 oder 12, wobei der Ringebalg ein Saugbalg ist und ein gasförimiges Medium ansaugt durch Auseinanderfalten des Ringebalges nur unter Schwerkraftwirkung und im Wesentlichen in Lotrichtung.

14. Verfahren zur Herstellung eines Ringebalgs nach einem der Ansprüche 1 bis 10 bzw. der Saugvorrichtung nach Anspruch 11 bis 13, wobei die Spannringe (10) auf die ausgedehnte Innenseite der Balghülle (1) aufgespritzt werden und sich unter Aushärten ausbilden.

15. Verfahren nach Anspruch 14, wobei die Balghülle (1) gegen die Innenwände eines runden oder ovalen Zylinders (21) durch einen Innendruck gepresst wird, bevor die Spannringe (10) auf die gestreckte Innenseite der Balghülle (1) aufgespritzt werden.
